# EUROPÄISCHE PATENTANMELDUNG

(11) **EP 2 517 737 A2**
(43) Veröffentlichungstag der Anmeldung: **31.10.2012**
(21) Anmeldenummer: 12165022.0
(22) Anmeldetag: 20.04.2012
(51) Int. Cl.: A61L 27/32, A61L 27/50, A61L 27/56

(54) **Implantat und Verfahren zur Herstellung einer Implantatoberfläche**

(30) Priorität: 21.04.2011 DE 102011018677
(71) Anmelder: ZL Microdent-Attachment GmbH & Co. KG, 58339 Breckerfeld (DE)
(72) Erfinder: Schreckenbach, Joachim, 09337 Hohenstein-Ernsthal (DE); Graf, Hans-Ludwig, 04451 Borsdorf (DE); Packheiser, Kai-Uwe, 58339 Breckerfeld (DE); Clostermann, Volkhard-Hagen, 58097 Hagen (DE); Kaludevovic, Milena, D-04109 Leipzig (DE)
(74) Vertreter: Dörner, Kötter & Kollegen

(57) **Zusammenfassung**

Die Erfindung betrifft ein Implantat, insbesondere zahnmedizinisches Implantat, mit einer eine heterogen strukturierten Implantatoberfläche, wobei die Implantatoberfläche (3) durch eine Schicht (2) gebildet ist, die stochastisch verteilte Noppencluster (6) aufweist, die aus Noppenstrukturen (4) bestehen. Die Erfindung betrifft weiterhin ein Verfahren zur Herstellung einer Schicht zur Bildung einer heterogen strukturierten Implantatoberfläche nach einem der vorgenannten Ansprüche, bei dem die Schicht (2) auf einem Titanimplantat in einem wässrigem Elektrolyt von 1,1 - 10,5 % NaOH, vorzugsweise 2,5 - 6,5 % NaOH unter anodischer Polarisation mittels Impulsspannung erzeugt wird.

## Beschreibung

Die Erfindung betrifft ein Implantat, insbesondere ein zahnmedizinisches Implantat, mit einer heterogen strukturierten Implantatoberfläche nach dem Oberbegriff des Patentanspruchs 1. Die Erfindung betrifft weiterhin ein Verfahren zur Herstellung einer Schicht zur Bildung einer heterogen strukturierten Implantatoberfläche nach dem Oberbegriff des Patentanspruchs 8.

Ein gegenwärtig immer noch ungelöstes Problem der Implantologie, insbesondere bei Dentalimplantaten, ist der Rückgang von Zahnfleisch - oder Knochengewebe. Dadurch kommt es zur Lockerung des Implantates bis zum Implantatverlust. Zusätzlich auftretende Infektionsprozesse können eine beabsichtigte Regeneration der Gewebe behindern. Ein weiterer damit verbundener Nachteil ist das Sichtbarwerden von metallischen Titanoberflächen oder schwarz-grauen Oberflächenbeschichtungen von partiell freigelegten Implantaten im Mundraum. Dies wird neben der Funktionsbeeinträchtigung als ästhetisch stark störend empfunden. Mit verschiedenen Ansätzen wird versucht, den Zielkonflikt zwischen hochbiokompatiblen Schichten und einer für den oralen Einsatz ästhetisch befriedigenden Oberflächenoptik zu lösen.

Als Mittel der Wahl wurden und werden Vereinzelt Dentalimplantate aus Zirkoniumoxid angesehen, da Zirkoniumoxid über eine hohe Biokompatibilität verfügt und gleichzeitig wegen seiner zahnfarbenen Optik den ästhetischen Anforderungen an helle, zahnähnliche Implantate erfüllt. Es hat sich aber in der Praxis als nachteilig gezeigt, dass derartige Vollkeramikimplantate eine glatte und wenig strukturierte Oberfläche besitzen, was zu einer deutlichen Reduzierung der Verankerung dieser Implantate im Kieferknochen führt. Versuche, dieses Problem durch spezielle Ausbrenntechniken von Spacern zu lösen, erwiesen sich als wenig zufrieden stellend.

Eine weitere Problematik von Zr-Vollkeramikimplantaten besteht in deren reduzierten mechanischen Bruchfestigkeit. Dabei kommt es mit zunehmender Einsatzdauer der Implantate infolge von Gittertransformationen zu Phasenumwandlungen des Zirkonoxides, welche verstärkt zur Riss- und Bruchbildung in der Keramik führen.

Weitere Lösungsansätze stellen PVD und PECVD Verfahren zur Implantatbeschichtung dar. Dabei werden auf den Implantatkörpern beispielsweise Niob, Tantal, Zirkonium oder Titan als Schicht aufgebracht und anschließend zur Oberflächenmodifizierung elektrochemisch, z.B. durch anodische Oxidation bearbeitet (DE 102006013115 A1). Nachteilig hierbei sind die geringe Schichthaftung und der hohe technische Aufwand zur Herstellung derselbigen.

In EP 1527790 A1 wird ein Implantat beschrieben, welche sowohl eine separate Weichgewebs- als auch eine Knochenkontaktfläche aufweist. Dabei kann die Weichgewebskontaktfläche durch verschiedene Verfahren so beschichtet werden, dass ein Durchschimmern von Metall im Weichgewebsteil ausgeschlossen wird. Hierbei sind aber insbesondere eine verringerte Biokompatibilität und zelluläre Reizungen im Übergangsbereich Weichgewebskontaktfläche/Knochenkontaktfläche zu berücksichtigen, da eine exakte Vorbestimmung einer sich variabel verhaltenden Übergangszone Weichgewebe/Knochen problematisch ist.

Weiterhin sind Beschichtungen mit Silikaten bekannt. Nachteilig sind hier, neben der geringen Biokompatibilität von Si-Oxiden, Mikrospannungen, die zum vorzeitigen Versagen dieser Schichten führen können (WO 01/74730, DE 102007013915 A1). In DE 102009035795 A1 wird eine strukturierte Implantatoberfläche beschrieben, die durch Thermolyse metallorganischer Verbindungen, die z.B. auch Indium (In) oder Thallium (TI) enthalten, gebildet. Derartige Überzüge sind kritisch zu betrachten, da z.B. Thallium-Verbindungen hoch toxisch sind und daher u.a. zur Nagerbekämpfung eingesetzt werden.

Hier will die Erfindung Abhilfe schaffen. Der Erfindung liegt die Aufgabe zu Grunde, Implantate mit hoher Biokompatibilität bei gleichzeitig hohen ästhetischen Ansprüchen bereitzustellen und die vorstehend beschriebenen Nachteile zu beseitigen. Ziel ist es, eine heterogen-mikrostrukturierte Implantatoberfläche mit heller Optik zu entwickeln.

Der Erfindung liegt weiterhin die Aufgabe zu Grunde, ein Verfahren zur Herstellung solcher Implantatoberflächen bereitzustellen. Überraschend wurde gefunden, dass mit Hilfe des Prozesses der anodischen Oxidation unter spezifischen Verfahrensbedingungen heterogene, mit noppen-ähnlichen Clustern mikrostrukturierte, helle Implantatoberflächen erzeugt werden können, die gleichzeitig eine hohe Biokompatibilität aufweisen. Diese Strukturen unterscheiden sich signifikant von den bisher beschriebenen Oberflächen, insbesondere von Oberflächen, die mittels anodischer Oxidation gebildet werden (DE 60019752 T2, WO 02/078759 A1, WO 00/72776 A1, US 4846837, DD 210607), welche mikroskopisch homogene Oberflächen aufweisen.

Die erfindungsgemäße Oberfläche zeichnet sich durch clusterförmig angeordnete Noppenstrukturen aus, welche unregelmäßig auf der Oberfläche angeordnet sind. Zwischen diesen Clustern befinden sich relativ ebene, schwach bzw. nanostukturierte Oberflächenbereiche, welche eine porige Struktur aufweisen. Die Porendurchmesser liegen hierbei unter 5 µm. Es wurde überraschend gefunden, dass derartige Strukturkombinationen vorteilhaft für den implantologischen Einsatz sind. Die Clusterstrukturen setzen sich aus einzelnen Noppen zusammen, die aus erschmolzenen Ti-Oxiden bestehen. Diese Noppen besitzen keine zentrale Pore. Die geometrische Grundform der Noppen ist kugelig bis zylindrisch, wobei das Verteilungsmaximum der geometrischen Noppenabmessungen zwischen 10 und 40 µm, vorzugsweise zwischen 18 und 32 µm liegt. Ein Noppencluster besteht aus 3 - 80 Noppenstukturen, vorzugsweise aus 10 bis 30 Noppenstrukturen. Das Verteilungsmaximum der Durchmesser solcher Noppencluster liegt zwischen 20 und 500 µm, bevorzugt zwischen 50 und 200 µm.

Die Abstände zwischen benachbarten Zentren der Noppencluster liegen zwischen 25 und 600 µm, bevorzugt zwischen 100 und 300 µm. Der Gesamtanteil an röntgenamorphen Schichtbestandteilen ist kleiner als 6% . Die Beschichtung enthält als kristalline Phase vorwiegend Rutil. Weiterhin enthält die Schicht monokline Na-Ti-Oxide der chemischen Zusammensetzung Na₂Ti₆O₁₃, diese sind überwiegend in den Noppenstrukturen konzentriert. Optisch zeigt sich die Oberfläche in einem elfenbeinähnlichen Weiß.

Die Erfindung stellt auch ein Verfahren zur Herstellung heterogen strukturierter Oberflächen bereit. Dabei wird in einem wässrigem Elektrolyt von 1,1 - 10,5 % NaOH, vorzugsweise 2,5 - 6,5 % NaOH unter anodischer Polarisation mittels Impulsspannung und Impulsspitzenspannungen von größer 210 V eine Schichtbildung vollzogen. In einem besonderen Ausführungsbeispiel wird die erfindungsgemäße Schicht einer zweiten elektrochemischen Behandlung unterzogen. Hierbei wird die aus dem ersten Verfahrensschritt ( Anodisation in NaOH) resultierende Beschichtung in einem Elektrolyten, der 0,2 mol/l Ca-Ionen und 0,4 mol/l Phospat-Ionen enthält, anodisch formiert. Dabei bilden sich zusätzliche Schichtbestandteile, welche Ca- und Phosphatverbindungen enthalten und damit zu einer weiteren Erhöhung der Biokompatibilität führen.

Andere Weiterbildungen und Ausgestaltungen der Erfindung sind in den übrigen Unteransprüchen angegeben. Ein Ausführungsbeispiel der Erfindung ist in der Zeichnung dargestellt und wird nachfolgend im Einzelnen beschrieben. Es zeigen:
- Fig. 1: Die Darstellung eines Titan-Implantats mit weißer anodischer Beschichtung;
- Fig. 2: die mikroskopische Darstellung der Oberfläche der Beschichtung des Implantats aus Figur 1;
- Fig. 3: eine weitere mikroskopische Darstellung der Oberfläche der Beschichtung des Implantats auf Figur 1;
- Fig. 4: die Darstellung der Noppenstruktur der Oberfläche der Beschichtung des Implantats aus Figur 1 in vergrößerter Ansicht;
- Fig. 5: die Darstellung der porigen Struktur der Clusterzwischenbereiche der Beschichtung des Implantats aus Figur 1 und
- Fig. 6: die schematische Darstellung der Clusterstrukturen eines erfindungsgemäßen Implantats
a) in der Draufsicht
b) im Querschnitt.

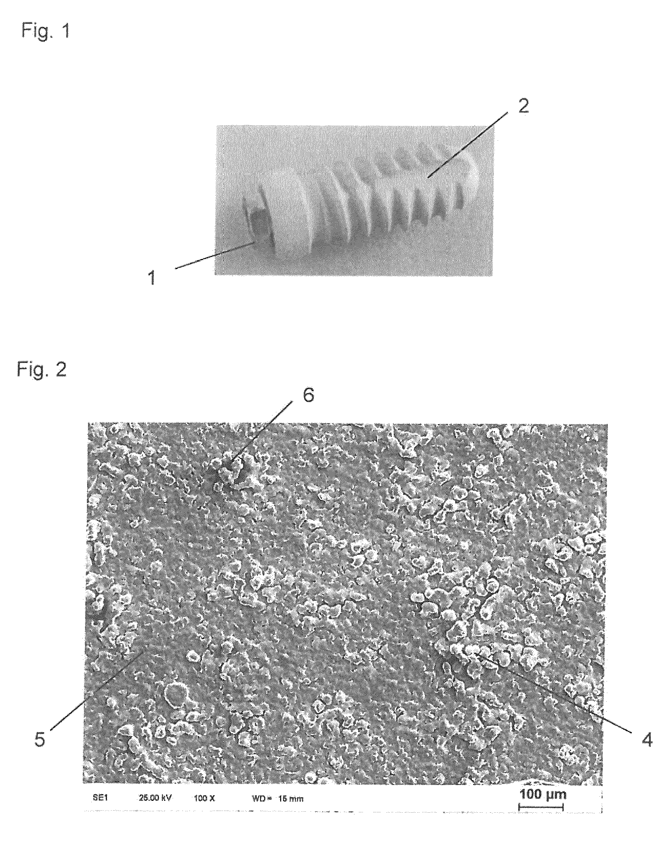

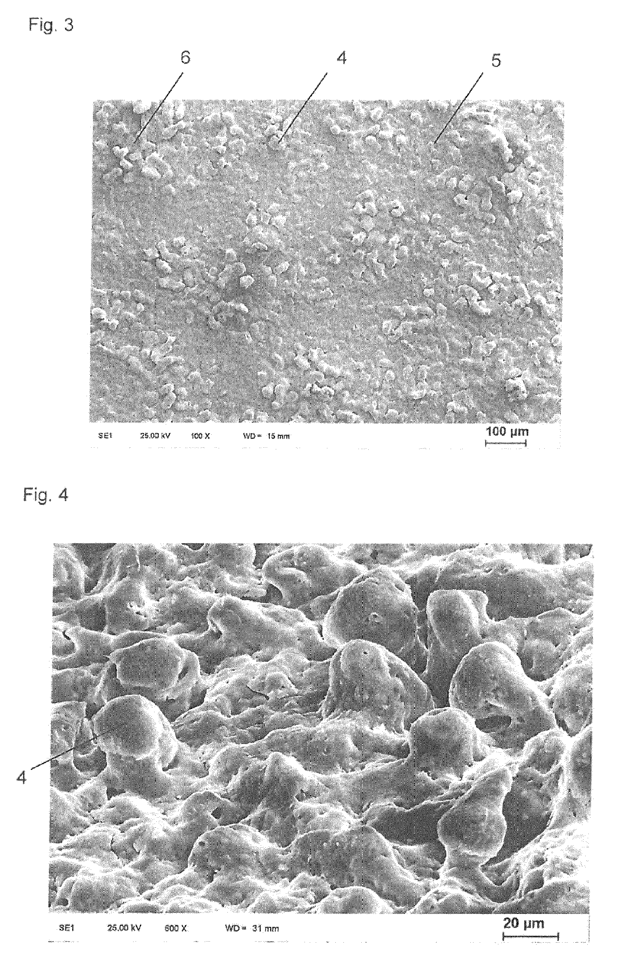

Das als Ausführungsbeispiel gewählte Implantat besteht im Wesentlichen aus einem Implantatkörper 1, der eine Beschichtung 2 aufweist. Die durch die Schicht 2 gebildete Implantatoberfläche 3 ist mikroskopisch vergrößert in den Figuren 2 und 3 dargestellt. Es sind deutlich clusterförmige Verteilung von noppenbildenden Ti-Oxid-Ausstülpungen (Noppen 4) zu erkennen, zwischen denen relativ ebene, glatte Oberflächenabschnitte 5 angeordnet sind.

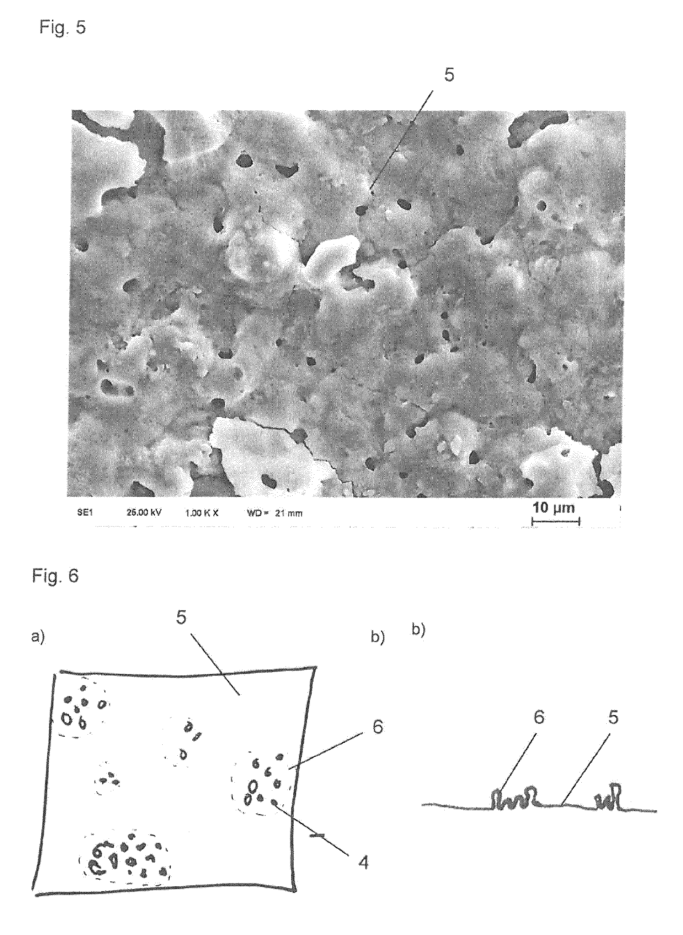

Figur 4 zeigt eine vergrößerte Ansicht der Noppenstruktur 41, in Figur 5 ist die porige Struktur der Clusterzwischenbereiche 5 dargestellt. Die Beschichtung 2 des Implantatkörpers 1 ist weiß ausgebildet und enthält Na-Ti-Oxid (Na₂Ti₆O₁₃), welches vorwiegend in den Noppen 4 konzentriert ist. Die Beschichtung 2 besteht im Wesentlichen aus Rutilkristallen und ist nicht röntgenamorph. Durch die Noppenansammlungen sind Clusterstrukturen gebildet, wobei zwischen den einzelnen Clustern 6 glatte Oberflächenabschnitte 5 angeordnet sind, wie in Figur 6 schematisch dargestellt. Der Durchmesser der Cluster 6 beträgt zwischen 50 und 200 µm, wobei jedes Cluster 6 etwa zehn bis dreißig Noppen 4 aufweist. Der Abstand der Noppenzentren beträgt etwa 100 bis 300 µm. Die Noppengrundform ist kugelich bis zylindrisch und weist Abmessungen zwischen 18 und 32 µm auf.

Das Ausführungsbeispiel ist keine sequentielle Beschichtung, also hier ohne Ca/P Elektrolyt. Zunächst wird hier eine Beschichtung durch anodische Oxidation in 3-5 % NaOH unter Impulsstrom erzeugt. Anschließend erfolgt eine elektrochemische Zweitbeschichtung in Ca/P-Elektrolyt, der 0,2 mol/l Ca-Ionen und 0,4 mol/l Phospat-Ionen enthält.

## Patentansprüche

1. Implantat, insbesondere zahnmedizinisches Implantat, mit einer eine heterogen strukturierten Implantatoberfläche, **dadurch gekennzeichnet, dass** die Implantatoberfläche (3) durch eine Schicht (2) gebildet ist, die stochastisch verteilte Noppencluster (6) aufweist, die aus Noppenstrukturen (41) bestehen.

2. Implantat nach Anspruch 1, **dadurch gekennzeichnet, dass** zwischen den Noppenclustern (6) porig strukturierte Bereiche angeordnet sind, die eine Porendurchmesser von kleiner 5 µm aufweisen.

3. Implantat nach Anspruch 1 oder 2, **dadurch gekennzeichnet, dass** das Verteilungsmaximum der geometrischen Noppenabmessungen zwischen 10 und 40 µm, vorzugsweise zwischen 18 und 32 µm liegt.

4. Implantat nach einem der vorgenannten Ansprüche, **dadurch gekennzeichnet, dass** wenigstens ein Noppencluster (6) aus 3 bis 80, bevorzugt 10 bis 30 Noppenstrukturen(41) besteht.

5. Implantat nach einem der vorgenannten Ansprüche, **dadurch gekennzeichnet, dass** das Verteilungsmaximum der Durchmesser der Noppencluster (6) zwischen 20 und 500 µm, bevorzugt zwischen 50 und 200 µm liegt.

6. Implantat nach einem der vorgenannten Ansprüche, **dadurch gekennzeichnet, dass** die Abstände zwischen benachbarten Zentren der Noppencluster (6) zwischen 25 und 600 µm, bevorzugt zwischen 100 und 300 µm liegen.

7. Implantat nach einem der vorgenannten Ansprüche, **dadurch gekennzeichnet, dass** die die Implantatoberfläche (3) bildende Schicht (2) monokline Na- Ti-Oxide der chemischen Zusammensetzung Na2Ti6O13 enthält, die sich überwiegend in den Noppenstrukturen (41) konzentrieren.

8. Verfahren zur Herstellung einer Schicht zur Bildung einer heterogen strukturierten Implantatoberfläche nach einem der vorgenannten Ansprüche, bei dem die Schicht (2) auf einem Titanimplantat in einem wässrigem Elektrolyt von 1,1 - 10,5 % NaOH, vorzugsweise 2,5 - 6,5 % NaOH unter anodischer Polarisation mittels Impulsspannung erzeugt wird.

9. Verfahren nach Anspruch 8, **dadurch gekennzeichnet, dass** die Impulsspannung mit Impulsspitzenspannungen von größer 210 V erzeugt wird.

10. Verfahren nach Anspruch 8 oder 9, **dadurch gekennzeichnet, dass** die erzeugte Schicht einer zweiten elektrochemischen Behandlung in einem Elektrolyten, der 0,2 mol/l Ca-Ionen und 0,4 mol/l Phospat-Ionen enthält, unterzogen wird.
